(19) Europäisches Patentamt / European Patent Office / Office européen des brevets

(11) **EP 3 680 293 B1**

(12) **EUROPEAN PATENT SPECIFICATION**

(45) Date of publication and mention of the grant of the patent:
**27.09.2023 Bulletin 2023/39**

(21) Application number: **18850271.0**

(22) Date of filing: **24.08.2018**

(51) International Patent Classification (IPC):
**A61Q 17/00** (2006.01)    **C08F 220/06** (2006.01)
**C08F 2/08** (2006.01)    **A61K 8/04** (2006.01)
**A61K 8/81** (2006.01)

(52) Cooperative Patent Classification (CPC):
(C-Sets available)
**A61K 8/042; A61K 8/8152; A61Q 17/005;**
**C08F 2/08; C08F 220/06; C08K 5/10; C08L 33/02;**
A61K 2800/48    (Cont.)

(86) International application number:
**PCT/JP2018/031291**

(87) International publication number:
**WO 2019/044680 (07.03.2019 Gazette 2019/10)**

(54) **CARBOXYL GROUP-CONTAINING POLYMER COMPOSITION AND METHOD FOR PRODUCING SAME**

CARBOXYLGRUPPEN-HALTIGE POLYMERZUSAMMENSETZUNG UND VERFAHREN ZU IHRER HERSTELLUNG

COMPOSITION DE POLYMÈRE CONTENANT UN GROUPE CARBOXYLE ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2017 JP 2017165400**

(43) Date of publication of application:
**15.07.2020 Bulletin 2020/29**

(73) Proprietor: **Sumitomo Seika Chemicals Co., Ltd.**
**Kako-gun, Hyogo 675-0145 (JP)**

(72) Inventors:
• **NISHIGUCHI, Satoshi**
  **Tokyo 102-0073 (JP)**
• **MURAKAMI, Ryosuke**
  **Himeji-shi**
  **Hyogo 672-8076 (JP)**
• **KIYOMOTO, Rie**
  **Himeji-shi**
  **Hyogo 672-8076 (JP)**
• **HASHIMOTO, Naoyuki**
  **Himeji-shi**
  **Hyogo 672-8076 (JP)**

(74) Representative: **Mewburn Ellis LLP**
**Aurora Building**
**Counterslip**
**Bristol BS1 6BX (GB)**

(56) References cited:
EP-A1- 3 205 694    WO-A1-2016/056591
WO-A1-2017/170576    WO-A1-2017/170577
WO-A1-2017/170577    JP-A- S61 223 010
JP-A- 2000 086 708    JP-A- 2011 105 833

(52) Cooperative Patent Classification (CPC): (Cont.)

C-Sets
**C08F 220/06, C08F 220/1818, C08F 220/1818,
C08F 220/1818, C08F 216/125**

**Description**

TECHNICAL FIELD

The present invention relates to a carboxyl group-containing polymer composition and a method for producing the same.

BACKGROUND ART

Conventionally, carboxyl group-containing polymers are used as thickeners for cosmetics, moisturizers for cataplasms, emulsifiers, suspension stabilizers, or gel bases for batteries and the like. When these carboxyl group-containing polymers are used for these purposes, the carboxyl group-containing polymers are, for example, added to water or the like to prepare homogeneous dispersions, and then neutralized with alkalis. In general, however, because the carboxyl group-containing polymers are fine powders, the carboxyl group-containing polymers are apt to form masses (unswollen lumps) when the carboxyl group-containing polymers are dispersed in water or the like. Once unswollen lumps are formed, a gel layer is formed on the unswollen lump surface, which slows down the rate of penetration of water into the unswollen lumps. This makes it difficult to obtain a homogeneous dispersion.

From the viewpoint of preventing the formation of unswollen lumps, it is known that, when a carboxyl group-containing polymer is prepared by polymerizing an α,β-unsaturated carboxylic acid and the like, a specific amount of at least one compound of a polyhydric alcohol fatty acid ester alkylene oxide adduct and a polyhydric alcohol fatty acid ester is added, and the polymerization is carried out to obtain a carboxyl group-containing polymer composition having excellent dispersibility in water (see Patent Documents 1-3).

PRIOR ART DOCUMENT

PATENT DOCUMENT

Patent Document 1: Japanese Patent Laid-Open Publication No. 2000-355614 Patent Document D2: EP 3205694 A1, Patent Document 3: Japanese patent Laid-Open Publication No. 2011-105833 SUMMARY OF THE INVENTION

PROBLEMS TO BE SOLVED BY THE INVENTION

There has recently been an increasing demand for carboxyl group-containing polymers as thickeners for antibacterial alcohol gels. However, the carboxyl group-containing polymer composition disclosed in Patent Document 1 has excellent dispersibility in water, but the carboxyl group-containing polymer composition has a problem of turbidity when an alcohol gel is prepared. As used herein, an "alcohol gel" refers to "a gel obtained by dispersing the carboxy group-containing polymer composition in an alcohol aqueous solution containing a total of 54 to 94% by mass of one or two or more alcohol components, and neutralizing the solution". In the present invention, an "alcohol gel A" whose a viscosity at 25°C and a light transmittance at a wavelength of 425 nm are measured, which will be described later, is included in the concept of the "alcohol gel".

The carboxyl group-containing polymer composition disclosed in Patent Document 1 may be unfavorable for purposes such as cosmetics because the odor of a solvent used during production is apt to remain.

It is a main object of the present invention to provide a carboxyl group-containing polymer composition which has little odor and excellent dispersibility in water, and which additionally has excellent transparency and excellent thickening properties for alcohol gels. Furthermore, it is another object of the present invention to provide a method for producing the carboxyl group-containing polymer composition.

MEANS FOR SOLVING THE PROBLEM

After thorough research to solve the aforementioned problem, the present inventors focused on the addition timing of a surfactant contained in a carboxyl group-containing polymer composition. As a result, the present inventors found that, in a method for producing a carboxyl group-containing polymer composition containing a carboxyl group-containing polymer (A) that is a copolymer of monomers containing an α,β-unsaturated carboxylic acid (a-1) and a compound (a-2) having at least two ethylenically unsaturated groups in its molecule; and a nonionic surfactant (B having an HLB value of 5 to 8), in the step of copolymerizing the monomers, the nonionic surfactant (B) is added into the system when a polymerization degree of the α,β-unsaturated carboxylic acid (a-1) reaches 5% or more to less than 70% (that is, when 5% or more to less than 70% of all the α,β-unsaturated carboxylic acid (a-1) used in the step of copolymerizing the monomers is copolymerized), whereby the obtained carboxyl group-containing polymer composition has little odor and excellent dispersibility in water, and additionally has excellent transparency and excellent thickening properties for alcohol gels. The present invention was accomplished by further conducting extensive research, based on this finding.

That is, the present invention provides aspects of the invention having the following configurations.

Item 1. A carboxyl group-containing polymer composition comprising: a carboxyl group-containing polymer (A) that is a copolymer of monomers containing an α,β-unsaturated carboxylic acid (a-1) and a compound (a-2) having at least two ethylenically unsaturated groups in its molecule; and a nonionic surfactant (B) having an HLB value of 5 to 8, wherein the carboxyl group-containing polymer composition is produced by a method comprising a step of copolymerizing the monomers, wherein the nonionic surfactant (B) is added into a system when a polymerization degree of the α, β-unsaturated carboxylic acid (a-1) reaches 5% or more to less than 70% in the step, and wherein an alcohol gel A prepared by the following preparation method has a viscosity of 5,000 mPa·s or more at 25°C, the alcohol gel A has a light transmittance of 95% or more at a wavelength of 425 nm, and the preparation method of the alcohol gel A includes homogeneously mixing 0.75 parts by mass of the carboxyl group-containing polymer composition, 51.70 parts by mass of ion exchange water, and 96.0 parts by mass of ethanol with each other, and then adding an ethanol solution of triethanolamine having a concentration of 50% by mass to the resulting mixture until a pH of the mixture reaches 7.0, followed by stirring until the mixture becomes homogeneous to prepare the alcohol gel A.

Item 2. The carboxyl group-containing polymer composition according to Item 1, wherein a content of the nonionic surfactant (B) is 0.5 to 9 parts by mass with respect to 100 parts by mass of the α,β-unsaturated carboxylic acid (a-1).

Item 3. A method for producing a carboxyl group-containing polymer composition containing a carboxyl group-containing polymer (A) that is a copolymer of monomers containing an α,β-unsaturated carboxylic acid (a-1) and a compound (a-2) having at least two ethylenically unsaturated groups in its molecule; and a nonionic surfactant (B) having an HLB value of 5 to 8, the method comprising a step of copolymerizing the monomers, wherein the nonionic surfactant (B) is added into a system when a polymerization degree of the α,β-unsaturated carboxylic acid (a-1) reaches 5% or more to less than 70% in the step.

Item 4. The method for producing a carboxyl group-containing polymer composition according to Item 4, wherein a content of the nonionic surfactant (B) is 0.5 to 9 parts by mass with respect to 100 parts by mass of the α,β-unsaturated carboxylic acid (a-1).

ADVANTAGES OF THE INVENTION

The preset invention can provide a carboxyl group-containing polymer composition which has little odor and excellent dispersibility in water, and which additionally has excellent transparency and excellent thickening properties for alcohol gels. The present invention can also provide a suitable production method for the carboxyl group-containing polymer composition.

EMBODIMENTS OF THE INVENTION

A carboxyl group-containing polymer composition according to the present invention contains: a carboxyl group-containing polymer (A) that is a copolymer of monomers containing an α,β-unsaturated carboxylic acid (a-1) and a compound (a-2) having at least two ethylenically unsaturated groups in its molecule; and a nonionic surfactant (B) having an HLB value of 5 to 8, wherein the carboxyl group-containing polymer composition is produced by a method comprising a step of copolymerizing the monomers, wherein the nonionic surfactant (B) is added into a system when a polymerization degree of the α, β-unsaturated carboxylic acid (a-1) reaches 5% or more to less than 70% in the step and , wherein an alcohol gel A prepared by the following preparation method has a viscosity of 5,000 mPa s or more at 25°C, and the alcohol gel A has a light transmittance of 95% or more at a wavelength of 425 nm;

(Preparation Method of Alcohol Gel A)

0.75 parts by mass of the carboxyl group-containing polymer composition, 51.70 parts by mass of ion exchange water, and 96.0 parts by mass of ethanol are homogeneously mixed with each other. Then, an ethanol solution of triethanolamine having a concentration of 50% by mass (an ethanol solution having a triethanolamine concentration of 50% by mass) is added to the resulting mixture until a pH of the mixture reaches 7.0, followed by stirring until the mixture becomes homogeneous, to prepare an alcohol gel A.

The carboxyl group-containing polymer composition of the present invention is suitably produced by, for example, the following method. That is, the carboxyl group-containing polymer composition of the present invention is suitably produced by a method for producing a carboxyl group-containing polymer composition containing a carboxyl group-containing polymer (A) that is a copolymer of monomers containing an α,β-unsaturated carboxylic acid (a-1) and a compound (a-2) having at least two ethylenically unsaturated groups in its molecule; and a nonionic surfactant (B) having an HLB value of 5 to 8, the method comprising a step of copolymerizing the monomers, wherein the nonionic surfactant (B) is added into a system when a polymerization degree of the α,β-unsaturated carboxylic acid (a-1) reaches 5% or more to less than 70% in the step.

Hereinafter, a carboxyl group-containing polymer composition of the present invention and a method for producing the same will be described in detail.

In the carboxyl group-containing polymer composition of the present invention, a carboxyl group-containing polymer (A) is a copolymer of monomers containing an α,β-unsaturated carboxylic acid (a-1) and a compound (a-2) having at least two ethylenically unsaturated groups in its molecule. By copolymerizing the monomers containing an α,β-unsaturated carboxylic acid (a-1) and a compound (a-2) having at least two ethylenically unsaturated groups in its molecule, the carboxyl group-containing polymer (A) is produced. That is, at least the α,β-unsaturated carboxylic acid (a-1) and the compound (a-2) having at least two ethylenically unsaturated groups in its molecule are used as monomers, and monomers including these components are copolymerized to produce the carboxyl group-containing polymer (A).

(α,β-Unsaturated Carboxylic Acid)

Examples of the α,β-unsaturated carboxylic acid (a-1) (may be referred to as a monomer (a-1)) include, but are not particularly limited to, olefinic unsaturated carboxylic acids having 3 to 5 carbon atoms such as acrylic acid, methacrylic acid, crotonic acid, maleic acid, itaconic acid, and fumaric acid. Among these α,β-unsaturated carboxylic acids (a-1), acrylic acid and methacrylic acid are suitably used, because they are inexpensive and readily available, and impart high transparency to a neutral viscous solution obtained using the resulting carboxyl group-containing polymer composition, and additionally have excellent transparency and excellent thickening properties for alcohol gels. These α,β-unsaturated carboxylic acids may be used alone or in combinations of two or more. As used herein, the "neutral viscous solution" refers to a solution prepared by dispersing the carboxyl group-containing polymer composition in water, and subsequently adjusting the dispersion to a pH of about 7 (pH=6 to 8) by adding a neutralizer such as an alkaline compound.

(Compound Having at Least Two Ethylenically Unsaturated Groups in Its Molecule)

Examples of the compound (a-2) having at least two ethylenically unsaturated groups in its molecule (compound (a-2) having two or more ethylenically unsaturated groups in its molecule, hereinafter, mentioned as a compound (a-2) having at least two ethylenically unsaturated groups) include, but are not particularly limited to, di-substituted or higher acrylic acid esters of polyols; di-substituted or higher methacrylic acid esters of polyols; di-substituted or higher allyl ethers of polyols; diallyl phthalate, triallyl phosphate, allyl methacrylate, tetraallyloxyethane, triallyl cyanurate, divinyl adipate, vinyl crotonate, 1,5-hexadiene, and divinylbenzene. Examples of the polyols include ethylene glycol, propylene glycol, polyoxyethylene glycol, polyoxypropylene glycol, glycerin, polyglycerin, trimethylolpropane, pentaerythritol, saccharose, and sorbitol. Among these compounds having at least two ethylenically unsaturated groups, pentaerythritol polyallyl ethers such as pentaerythritol diallyl ether, pentaerythritol triallyl ether, and pentaerythritol tetraallyl ether, tetraallyloxyethane, triallyl phosphate, and polyallylsaccharose are suitably used from the viewpoint that they facilitate the viscosity adjustment of a neutral viscous solution obtained using the resulting carboxyl group-containing polymer composition, and additionally have excellent transparency and excellent thickening properties for alcohol gels. These compounds (a-2) having at least two ethylenically unsaturated groups may be used alone or in combinations of two or more.

The lower limit of the proportion of the compound (a-2) having at least two ethylenically unsaturated groups is preferably 0.01 parts by mass or more, and more preferably 0.05 parts by mass or more, with respect to 100 parts by mass of the α,β-unsaturated carboxylic acid. The upper limit of the proportion of the compound (a-2) having at least two ethylenically unsaturated group is preferably 10 parts by mass or less, and more preferably 3 parts by mass or less. The proportion of the compound (a-2) having at least two ethylenically unsaturated groups is preferably within a range of 0.01 to 10 parts by mass, 0.01 to 3 parts by mass, 0.05 to 10 parts by mass, or 0.05 to 3 parts by mass. When the proportion of the compound (a-2) having at least two ethylenically unsaturated groups is 0.01 parts by mass or more, high transparency and high thickening properties for alcohol gels obtained using the resulting carboxyl group-containing polymer composition can be suitably expressed. When the amount of the compound having at least two ethylenically unsaturated groups used is 10 parts by mass or less, the carboxyl group-containing polymer composition having excellent transparency and excellent thickening properties for alcohol gels can be suitably provided. In the present invention, "100 parts by mass of the α,β-unsaturated carboxylic acid" refers to the entire amount of the α,β-unsaturated carboxylic acid (a-1).

(α,β-Unsaturated Compound)

As a monomer constituting the carboxyl group-containing polymer (A), an α,β-unsaturated compound (a-3) and the like may be used from the viewpoint of providing the carboxyl group-containing polymer composition suitably having excellent transparency and excellent thickening properties for alcohol gels.

The α,β-unsaturated compound (a-3) is not particularly limited as long as it differs from the α,β-unsaturated carboxylic acid (a-1), and examples include acrylic acid esters such as methyl acrylate, ethyl acrylate, isopropyl acrylate, butyl acrylate, octyl acrylate, 2-ethyl acrylate, decyl acrylate, lauroyl acrylate, stearyl acrylate, and glycidyl acrylate; methacrylic acid esters corresponding to the above-described acrylic acid esters; glycidyl ethers such as vinyl glycidyl ether, isopropenyl glycidyl ether, allyl glycidyl ether, and butenyl glycidyl ether; acrylamides such as acrylamide, N-methylacrylamide, N-ethylacrylamide, and N-t-butylacrylamide; methacrylamides corresponding to the acrylamides; and vinyl esters such as vinyl acetate, vinyl propionate, and vinyl benzoate. Among these α,β-unsaturated compounds (a-3), acrylic acid esters and methacrylic acid esters are suitably used. In particular, acrylic acid esters and methacrylic acid esters having linear or branched alkyl groups with 12 to 40 (particularly 15 to 30) carbon atoms are preferably used, and stearyl methacrylate, eicosanyl methacrylate, behenyl methacrylate, and tetracosanyl methacrylate are more preferably used. These α,β-unsaturated compounds (a-3) may be used alone or in combinations of two or more. As the acrylic acid ester or the methacrylic acid ester, a product such as trade name BLEMMER VMA70 manufactured by NOF Corporation, for example, may be used.

The lower limit of the proportion of the α,β-unsaturated compound (a-3) is preferably 0.1 parts by mass or more, and more preferably 1 part by mass or more, with respect to 100 parts by mass of the α,β-unsaturated carboxylic acid (a-1). The upper limit of the proportion of the α,β-unsaturated compound (a-3) is preferably 20 parts by mass or less, and more preferably 10 parts by mass or less. The proportion of the α,β-unsaturated compound (a-3) is preferably within a range of 0.1 to 20 parts by mass, 0.1 to 10 parts by mass, 1 to 20 parts by mass, or 1 to 10 parts by mass. When the proportion of the α,β-unsaturated compound (a-3) is 0.1 parts by mass or more, high transparency and high thickening properties for alcohol gels obtained using the resulting carboxyl group-containing polymer composition can be suitably expressed. When the amount of the α,β-unsaturated compound (a-3) used is 20 parts by mass or less, the carboxyl group-containing polymer composition having excellent transparency and excellent thickening properties for alcohol gels can be suitably provided.

In the present invention, although the total proportion of the α,β-unsaturated carboxylic acid (a-1), the compound (a-2) having at least two ethylenically unsaturated groups, and the α,β-unsaturated compound (a-3) in the monomers constituting the carboxyl group-containing polymer (A) is not particularly limited, the total proportion is, for example, preferably 50% by mass or more, more preferably about 80 to 100% by mass, still more preferably about 90 to 100% by mass, and particularly preferably about 95 to 100% by mass. The total proportion may be substantially 100% by mass. Although the proportion of the α,β-unsaturated carboxylic acid (a-1) in the monomers constituting the carboxyl group-containing polymer (A) is not particularly limited, the proportion is, for example, preferably 95% by mass or more, more preferably about 96 to 99% by mass, and still more preferably about 97 to 98% by mass.

The method for copolymerizing these monomers is not particularly limited, and a known or conventionally used method can be applied. Specifically, the copolymerization can be carried out using, for example, any of the following methods (1) to (3):

method (1): the α,β-unsaturated carboxylic acid (a-1) and the compound (a-2) having at least two ethylenically unsaturated groups (as well as optionally the α,β-unsaturated compound (a-3)) are allowed to coexist from the beginning of polymerization (for example, the entire amounts of the respective components are allowed to coexist), and the copolymerization is carried out;

method (2): the compound (a-2) having at least two ethylenically unsaturated groups is continuously added to the α,β-unsaturated carboxylic acid (a-1), and the polymerization is carried out simultaneously, wherein when the α,β-unsaturated compound (a-3) is used, the method for subjecting the compound to polymerization is not particularly limited, and, for example, the α,β-unsaturated compound (a-3) may be allowed to coexist with the α,β-unsaturated carboxylic acid (a-1) in advance, or may be continuously added like the compound (a-2) having at least two ethylenically unsaturated groups; and

method (3): the α,β-unsaturated carboxylic acid (a-1) and the compound (a-2) having at least two ethylenically unsaturated groups are continuously added, and the polymerization is carried out simultaneously, wherein when the α,β-unsaturated compound (a-3) is used, the method for subjecting the compound to polymerization is not particularly limited, and, for example, the α,β-unsaturated compound (a-3) may be introduced into the polymerization system in advance, or may be continuously added like the α,β-unsaturated carboxylic acid (a-1) and the compound (a-2) having at least two ethylenically unsaturated groups.

By way of example, the method (1) will be specifically described. In the method (1), desired amounts of the

$\alpha,\beta$-unsaturated carboxylic acid (a-1), the compound (a-2) having at least two ethylenically unsaturated groups, a radical polymerization initiator, and an inert solvent are weighed out in advance, and introduced into a reaction vessel equipped with a stirrer, a thermometer, a nitrogen gas inlet tube, and a condenser tube, for example.

Next, the contents in the reaction vessel are stirred and mixed to provide a homogeneous composition. Then, nitrogen gas is blown into the contents to remove oxygen gas contained in the upper space of the reaction vessel and dissolved oxygen dissolved in the contents. The polymerization reaction can be performed by heating to 20 to 120°C, preferably 30 to 90°C, in a warm bath or the like. The polymerization reaction is typically completed in 2 to 10 hours.

In the entire amount of the $\alpha,\beta$-unsaturated carboxylic acid (a-1), the compound (a-2) having at least two ethylenically unsaturated groups, optionally the $\alpha,\beta$-unsaturated compound (a-3), the radical polymerization initiator, and the inert solvent to be introduced, the total amount of the $\alpha,\beta$-unsaturated carboxylic acid (a-1) and the compound (a-2) having at least two ethylenically unsaturated groups to be introduced (when the $\alpha,\beta$-unsaturated compound (a-3) is used, the total proportion of the $\alpha,\beta$-unsaturated carboxylic acid (a-1), the compound (a-2) having at least two ethylenically unsaturated groups, and the $\alpha,\beta$-unsaturated compound (a-3)) is 1% by mass or more, and preferably 5% by mass or more, from the viewpoint of increasing the volume efficiency to improve the productivity, and is 30% by mass or less, and preferably 25% by mass or less, from the viewpoint of avoiding an increase in the viscosity of the slurry due to significant precipitation of the polymer along with the progress of the polymerization reaction, to cause the reaction to proceed smoothly. The total amount to be introduced is preferably 1 to 30% by mass, and more preferably 5 to 25% by mass.

The inert solvent for producing the carboxyl group-containing polymer (A) is not particularly limited as long as it is a solvent that dissolves the $\alpha,\beta$-unsaturated carboxylic acid (a-1), the compound (a-2) having at least two ethylenically unsaturated groups, and the $\alpha,\beta$-unsaturated compound (a-3), but does not dissolve the resulting carboxyl group-containing polymer composition. Preferable specific examples of the inert solvent include optionally halogen-substituted aliphatic hydrocarbons having 2 to 8 carbon atoms, such as ethylene dichloride, normal pentane, normal hexane, isohexane, normal heptane, normal octane, and isooctane; alicyclic hydrocarbons having 5 to 7 carbon atoms, such as cyclopentane, methylcyclopentane, cyclohexane, and methylcyclohexane; optionally halogen-substituted aromatic hydrocarbons, such as benzene, toluene, xylene, and chlorobenzene; acetic acid alkyl esters such as ethyl acetate and isopropyl acetate; and ketone-based compounds such as methyl ethyl ketone and methyl isobutyl ketone. These inert solvents may be used alone or in combinations of two or more. Among these inert solvents, ethylene dichloride, normal hexane, cyclohexane, normal heptane, and ethyl acetate are preferable from the viewpoint that they are stable in quality and readily available.

The type of the radical polymerization initiator is not particularly limited. Specific examples of the radical polymerization initiator include $\alpha,\alpha'$-azoisobutyronitrile, 2,2'-azobis-2,4-dimethylvaleronitrile, dimethyl-2,2'-azobisisobutylate, 2,2'-azobis(methyl isobutyrate), benzoyl peroxide, lauroyl peroxide, cumene hydroperoxide, and tert-butyl hydroperoxide. These radical polymerization initiators may be used alone or in combinations of two or more.

The amount of the radical polymerization initiator cannot be unequivocally determined since it varies depending on the type and the reaction temperature and the like. Typically, however, from the viewpoint of increasing the rate of the polymerization reaction, the amount of the radical polymerization initiator is preferably 0.1% by mass or more, and more preferably 0.3% by mass or more, based on the total amount of the $\alpha,\beta$-unsaturated carboxylic acid (a-1), the compound (a-2) having at least two ethylenically unsaturated groups, and the $\alpha,\beta$-unsaturated compound (a-3) that are used as monomers of the carboxyl group-containing polymer (A), and is preferably 10% by mass or less, and more preferably 3% by mass or less, in order to allow removal of heat during the polymerization reaction to be facilitated. The amount of the radical polymerization initiator is preferably 0.1 to 10% by mass, and more preferably 0.3 to 3% by mass.

It is preferable to remove oxygen from the reaction system in advance, because the presence of oxygen during the reaction adversely affects the reaction. Thus, the atmosphere during the reaction is preferably an inert gas atmosphere such as nitrogen gas or argon gas, for example, from the viewpoint of avoiding the influence of oxygen.

In the production method of the present invention, in the step of copolymerizing the monomers, the nonionic surfactant (B) is added into the system when the polymerization degree of the $\alpha,\beta$-unsaturated carboxylic acid (a-1) has reached 5% or more to less than 70%. That is, the nonionic surfactant (B) is added into the system in a state where the copolymerization of the $\alpha,\beta$-unsaturated carboxylic acid (a-1) has not yet completed (the polymerization degree is 5% or more and less than 70%). In the production method of the present invention, the nonionic surfactant (B) is added at the specific timing, whereby a carboxyl group-containing polymer composition can be produced that is excellent in all of the following: dispersibility in water, and transparency and thickening properties for alcohol gels. As long as the nonionic surfactant (B) is added at least when the polymerization degree is 5% or more to less than 70%, a similar effect is achieved even if the nonionic surfactant (B) is separately added when the polymerization degree is less than 5% or more than 80%.

In the production method of the present invention, the polymerization degree in the copolymerization step can be confirmed using the method described in Examples.

(Nonionic Surfactant)

The nonionic surfactant (B) is composed of a hydrophilic moiety and a hydrophobic moiety. Examples of the hydrophobic moiety include a polyhydric alcohol fatty acid ester and an addition polymer of a hydroxy fatty acid.

From the viewpoint of obtaining a carboxyl group-containing polymer composition which has little odor and excellent dispersibility in water, and which additionally has excellent transparency and excellent thickening properties for alcohol gels, suitable examples of the polyhydric alcohol moiety of the polyhydric alcohol fatty acid ester include glycerin, polyglycerin, trimethylpropanol, sorbitol, and sorbitan. The polyhydric alcohol residue of the nonionic surfactant (B) is preferably one derived from any of these polyhydric alcohols. These polyhydric alcohol moieties may be present alone or in combinations of two or more.

Suitable examples of the fatty acid moiety of the polyhydric alcohol fatty acid ester include stearic acid, isostearic acid, and palmitic acid. That is, the fatty acid residue of the nonionic surfactant (B) is preferably one derived from any of these fatty acids. These fatty acid moieties may be present alone or in combinations of two or more.

Examples of the polyhydric alcohol fatty acid ester in the hydrophobic moiety of the nonionic surfactant (B) include stearic acid esters, isostearic acid esters, palmitic acid esters, and hydrogenated castor oil derivatives.

Suitable examples of the addition polymer of the hydroxy fatty acid in the hydrophobic moiety of the nonionic surfactant (B) include addition polymers of 2-hydroxypalmitic acid, 16-hydroxypalmitic acid, and 12-hydroxystearic acid.

Examples of the hydrophilic moiety of the nonionic surfactant (B) include carboxy, hydroxy, amino, sulfonyl, and ether groups. The ether group in the hydrophilic moiety of the nonionic surfactant (B) may be an oxyalkylene chain such as polyoxyalkylene.

Suitable examples of the oxyalkylene chain in the nonionic surfactant (B) include an oxyalkylene group represented by the following formula:

$$\text{Formula:} \qquad -(CH_2\text{-}CHR^1\text{-}O)n-$$

-wherein $R^1$ represents a hydrogen atom, a methyl group, or an ethyl group; and n represents an integer from 1 to 100; and when n is an integer of 2 or more, a plurality of $R^1$s may each be the same or different.

From the viewpoint of obtaining a carboxyl group-containing polymer composition which has little odor and excellent dispersibility in water, and which additionally has excellent transparency and excellent thickening properties for alcohol gels, the nonionic surfactant (B) is preferably a polyhydric alcohol fatty acid ester alkylene oxide adduct. The polyhydric alcohol fatty acid ester alkylene oxide adduct is a compound in which an alkylene oxide is added to a fatty acid ester of a polyhydric alcohol. The polyhydric alcohol fatty acid ester alkylene oxide adduct serves as an emulsifier.

Suitable examples of the polyhydric alcohol moiety of the polyhydric alcohol fatty acid ester alkylene oxide adduct include glycerin, polyglycerin, trimethylpropanol, sorbitol, and sorbitan, as described above. That is, the polyhydric alcohol residue of the polyhydric alcohol fatty acid ester alkylene oxide adduct is preferably one derived from any of these polyhydric alcohols. These polyhydric alcohol moieties may be present alone or in combinations of two or more.

Suitable examples of the fatty acid moiety of the polyhydric alcohol fatty acid ester alkylene oxide adduct include stearic acid, isostearic acid, and palmitic acid, as described above. That is, the fatty acid residue of the polyhydric alcohol fatty acid ester alkylene oxide adduct is preferably one derived from any of these fatty acids. These fatty acid moieties may be present alone or in combinations of two or more.

As described above, examples of the polyhydric alcohol fatty acid ester of the polyhydric alcohol fatty acid ester alkylene oxide adduct include stearic acid esters, isostearic acid esters, palmitic acid esters, and hydrogenated castor oil derivatives.

Suitable examples of the oxyalkylene chain in the polyhydric alcohol fatty acid ester alkylene oxide adduct include an oxyalkylene group represented by the above general formula (I).

From the viewpoint of obtaining a carboxyl group-containing polymer composition which has little odor and excellent dispersibility in water, and which additionally has excellent transparency and excellent thickening properties for alcohol gels, the lower limit of the HLB of the nonionic surfactant (B) is 5 or more, and the upper limit of the HLB of the nonionic surfactant (B) is 8 or less. The HLB of the nonionic surfactant (B) is within a range of 5 to 8.

From the viewpoint of obtaining a carboxyl group-containing polymer composition which has little odor and excellent dispersibility in water, and which additionally has excellent transparency and excellent thickening properties for alcohol gels, preferable specific examples of the nonionic surfactant (B) include polyoxyethylene glyceryl triisostearate, polyoxyethylene (30) hydrogenated castor oil triisostearate, polyoxyethylene hydrogenated castor oil derivatives, polyoxyethylene glycerin fatty acid esters, and polyoxyethylene trimethylol tristearate. Specific examples of polyoxyethylene glycerin fatty acid esters include polyoxyethylene glyceryl monostearate, polyoxyethylene glyceryl distearate, polyoxyethylene glyceryl tristearate, polyoxyethylene glyceryl isostearate, polyoxyethylene glyceryl diisostearate, polyoxyethylene glyceryl triisostearate, a block copolymer of 2-hydroxypalmitic acid and alkylene glycol, a block copolymer of 16-hydroxypalmitic acid and alkylene glycol, and a block copolymer of 12-hydroxystearic acid and alkylene glycol.

When a hydrogenated castor oil derivative is used as the polyhydric alcohol fatty acid ester, preferable specific examples of the polyhydric alcohol fatty acid ester alkylene oxide adduct include polyoxyethylene hydrogenated castor oil, polyoxyethylene hydrogenated castor oil laurate, polyoxyethylene hydrogenated castor oil isostearate, and polyoxyethylene hydrogenated castor oil triisostearate.

Among the polyhydric alcohol fatty acid ester alkylene oxide adducts, from the viewpoint of obtaining a carboxyl group-containing polymer composition which has little odor and excellent dispersibility in water, and which additionally has excellent transparency and excellent thickening properties for alcohol gels, polyoxyethylene hydrogenated castor oil, polyoxyethylene hydrogenated castor oil isostearate, polyoxyethylene hydrogenated castor oil triisostearate, and polyoxyethylene sorbitol tetraoleate are preferable.

From the viewpoints of improving the dispersibility of the resulting carboxyl group-containing polymer composition in water, and obtaining the carboxyl group-containing polymer composition which has little odor, and which additionally has excellent transparency and excellent thickening properties for alcohol gels, the content of the nonionic surfactant (B) (that is, the amount of the nonionic surfactant (B) to be added when the polymerization degree is 5% or more and less than 70%) is preferably 0.5 parts by mass or more, and more preferably 3 parts by mass or more, with respect to 100 parts by mass of the $\alpha,\beta$-unsaturated carboxylic acid (a-1). From the viewpoint of obtaining a carboxyl group-containing polymer composition which has little odor and excellent dispersibility in water, and which additionally has excellent transparency and excellent thickening properties for alcohol gels, the content of the nonionic surfactant (B) is preferably 9 parts by mass or less, and more preferably 7 parts by mass or less, with respect to 100 parts by mass of the $\alpha,\beta$-unsaturated carboxylic acid (a-1). That is, the content of the nonionic surfactant (B) is preferably 0.5 to 9 parts by mass, 0.5 to 7 parts by mass, 3 to 9 parts by mass, or 3 to 7 parts by mass, with respect to 100 parts by mass of the $\alpha,\beta$-unsaturated carboxylic acid (a-1).

The method for producing a carboxyl group-containing polymer composition of the present invention can be carried out in accordance with, for example, the aspects of the following methods (1A) and (2A):

Method (1A): the $\alpha,\beta$-unsaturated carboxylic acid (a-1) and the compound (a-2) having at least two ethylenically unsaturated groups (as well as optionally the $\alpha,\beta$-unsaturated compound (a-3)) are introduced into a reaction vessel in advance; these monomers are copolymerized; and when the polymerization degree has reached 5% or more and less than 70%, the nonionic surfactant (B) is added into the slurry in the system.

Method (2A): the $\alpha,\beta$-unsaturated carboxylic acid (a-1) and the compound (a-2) having at least two ethylenically unsaturated groups are each continuously added into a reaction vessel, and the copolymerization is carried out simultaneously; and when the polymerization degree has reached 5 or more and less than 70%, the nonionic surfactant (B) is added into the slurry in the system.

When the $\alpha,\beta$-unsaturated compound (a-3) is used in the method (2A), the method for subjecting the compound to polymerization is not particularly limited, and, for example, the $\alpha,\beta$-unsaturated compound (a-3) may be introduced into the reaction vessel in advance, or may be continuously added into the reaction vessel like the $\alpha,\beta$-unsaturated carboxylic acid (a-1) and the compound (a-2) having at least two ethylenically unsaturated groups.

For example, in the method (1A), desired amounts of the $\alpha,\beta$-unsaturated carboxylic acid (a-1), the compound (a-2) having at least two ethylenically unsaturated groups, optionally the $\alpha,\beta$-unsaturated compound (a-3), a radical polymerization initiator, and an inert solvent are weighed out in advance, and introduced into a reaction vessel equipped with a stirrer, a thermometer, a nitrogen gas inlet tube, and a condenser tube. The contents in the reaction vessel are mixed with stirring to give a homogeneous composition, and then nitrogen gas is blown into the contents to remove oxygen gas contained in the upper space of the reaction vessel and dissolved oxygen dissolved in the contents. The polymerization reaction can be performed by heating to 20 to 120°C, preferably 30 to 90°C, in a warm bath or the like. The polymerization reaction is typically completed in 2 to 10 hours. Next, after the polymerization degree is confirmed to have reached 5% or more and less than 70%, the nonionic surfactant (B) is diluted with an inert solvent and added into the slurry in the system. The mixture is stirred to homogeneity. Thereafter, the inert solvent is distilled off from the reaction solution by heating under reduced pressure or normal pressure. In this manner, a carboxyl group-containing polymer composition can be obtained as a white fine powder.

Thus, a carboxyl group-containing polymer composition of the present invention can be suitably obtained. The carboxyl group-containing polymer composition of the present invention has little odor, and is excellent in all of the following: dispersibility in water, and transparency and thickening properties for alcohol gels. A neutral viscous solution can be obtained by, for example, dispersing the carboxyl group-containing polymer composition in water, and subsequently adjusting the pH of the aqueous solution to about 7 (pH = 6 to 8) by adding an alkaline component such as sodium hydroxide or triethanolamine. A neutralized alcohol gel can be obtained by dispersing the carboxy group-containing polymer composition in an alcohol aqueous solution containing a total of 54 to 94% by mass of alcohol, and subsequently adjusting the pH of the aqueous solution to about 7 (pH = 6 to 8) by adding an alkaline component such as sodium hydroxide or triethanolamine.

The carboxyl group-containing polymer composition of the present invention preferably has a residual solvent amount of 1000 ppm or less, and more preferably 750 ppm or less. The solvent remaining in the carboxyl group-containing polymer composition is a solvent used when monomers are copolymerized. The solvent is an inert solvent such as ethylene dichloride, normal hexane, cyclohexane, normal heptane, or ethyl acetate, as described above.

EXAMPLES

The present invention will be hereinafter described in detail with reference to Examples and Comparative Examples. However, the present invention is not limited to Examples.

[Evaluation Methods]

Various characteristics of carboxyl group-containing polymer compositions obtained in Examples and Comparative Examples were measured and evaluated using the following methods. The HLB values of nonionic surfactants are catalog values.

(1) Dispersion Time in Water

In a 200 mL (milliliter) volume beaker, 100 g of ion exchange water is placed, and the temperature of the ion exchange water is adjusted to 25°C. Into this beaker, 3.0 g of a carboxyl group-containing polymer composition is added all at once under non-stirring conditions, and the swollen state of the carboxyl group-containing polymer composition is visually observed to measure the time (min) required until the carboxyl group-containing polymer composition becomes completely wet without any dry portions (referred to as the "dispersion time"). When the time required for swelling is 30 minutes or shorter, the dispersibility can be determined to be excellent.

(2) Viscosity and Light Transmittance of Alcohol Gel A

In a 200 mL (milliliter) volume beaker, 51.7 g of ion exchange water and 96.0 g of ethanol are placed, and 0.75 g of a carboxy group-containing polymer composition is added with stirring. After the components could be homogeneously dispersed, stirring was stopped, an ethanol solution of triethanolamine having a concentration of 50% by mass (an ethanol solution having a triethanolamine concentration of 50% by weight) was added to give a pH of 7.0, and the gel was stirred to homogeneity with a hand mixer or the like, to obtain an alcohol gel. The beaker containing the alcohol gel was immersed in a thermostat at 25°C, and then the viscosity and light transmittance of the alcohol gel were measured. The viscosity of the alcohol gel was measured using a viscometer manufactured by Brookfield (model number: DV1MRVTJ0) at 20 rpm with spindle No. 5. When the viscosity of the alcohol gel was 5000 mPa·s or more, the viscosity was determined to be high. As for the light transmittance of the alcohol gel, the transmittance of light at a wavelength of 425 nm was measured using a spectrophotometer manufactured by Shimadzu Corporation (model number: UV-3150). When the light transmittance was 95% or more, the transparency was determined to be high.

(3) Polymerization Degree

The polymerization degree was calculated based on the expression shown below. The amount of the unreacted monomer (a-1) refers to the total amount of the monomer (a-1) that has not been added into the system at that time and the monomer (a-1) that is present in an unreacted state in the system, out of the entire amount of the monomer (a-1) used in the polymerization reaction. In the expression, each amount of the monomer (a-1) is based on the weight. The amount of the unreacted monomer (a-1) was measured by using liquid chromatography such as high performance liquid chromatography.

[Expression 1]

$$\text{Polymerization degree of monomer (a-1) [\%]} = \frac{\text{Total amount of monomer (a-1) used for polymerization reaction - amount of unreacted monomer (a-1)}}{\text{Total amount of monomer (a-1) used for polymerization reaction}} \times 100$$

(4) Odor

(4-1) Monitor Evaluation

Each of five monitors had a smell of each of the carboxy group-containing polymer compositions, and the carboxy group-containing polymer compositions were evaluated at the following five levels for odor. The average of the evaluations of the five monitors was taken as the score of the monitor evaluation of the odor of the carboxyl group-containing polymer composition. If the average score is 3 or more, the odor can be determined to be little.

1: feel a very potent smell
2: feel a potent smell
3: feel a smell in a degree of concern
4: feel a little smell but ignore the smell
5: odorless

(4-2) Amount of Residual Hexane

The amount of residual hexane of the carboxy group-containing polymer composition was calculated by the following formula.

[Expression 2]

$$\text{Amount of residual hexane [ppm]} \quad = \frac{\text{Amount of hexane in polymer [g]}}{\text{Amount of polymer [g]}} = \times 10^5$$

The amount of hexane in the carboxyl group-containing polymer composition was measured using gas chromatography. When the amount of residual hexane is less than 1000 ppm, the odor of hexane is less likely to be felt from the carboxyl group-containing polymer composition, so that the odor can be determined to be little.

(Example 1)

Into a 500 mL (milliliter) volume four-necked flask equipped with a stirrer, a thermometer, a nitrogen inlet tube, and a condenser tube were introduced 40 g of acrylic acid; 0.88 g of BLEMMER VMA70 (manufactured by NOF Corporation; a mixture containing 10 to 20 parts by mass of stearyl methacrylate, 10 to 20 parts by mass of eicosanyl methacrylate, 59 to 80 parts by mass of behenyl methacrylate, and 1% by mass or less of tetracosanyl methacrylate) as a (meth)acrylic acid alkyl ester in which the alkyl group has 18 to 24 carbon atoms; 0.20 g of pentaerythritol tetraallyl ether as the compound (a-2) having at least two ethylenically unsaturated groups; 0.116 g of 2,2'-azobis(methyl isobutyrate) as a radical polymerization initiator; and 230.9 g of normal hexane as a reaction solvent. Subsequently, the solution was homogeneously mixed with stirring, and then nitrogen gas was blown into the solution to remove oxygen present in upper space of the reaction vessel (four-necked flask), the raw materials, and the reaction solvent. Next, the contents were heated to 60 to 65°C in a nitrogen atmosphere. Thereafter, the contents were held at 60 to 65°C for 3 hours. When the polymerization degree reached 54% (when the contents were held at 60 to 65°C for about 1 hour), a solution of 2.0 g of a block copolymer of 12-hydroxystearic acid and polyoxyethylene (Hypermer B246, manufactured by Croda) as the nonionic surfactant (B) in 2.0 g of normal hexane was added into the reaction vessel. Thereafter, the resulting slurry was heated to 100°C to distill off the normal hexane, and the product was further dried under reduced pressure at 115°C and 10 mmHg for 8 hours, thereby obtaining 38 g of a carboxyl group-containing polymer composition as a white fine powder.

(Example 2)

Following the procedure of Example 1, 38 g of a carboxyl group-containing polymer composition was obtained as a white fine powder in the same manner as in Example 1 except that the nonionic surfactant (B) was changed to polyoxyethylene (30) hydrogenated castor oil triisostearate (EMALEX RWIS-330 manufactured by Nihon Emulsion Co., Ltd.). The polymerization degree when the nonionic surfactant was added was 50%.

(Example 3)

Following the procedure of Example 1, 38 g of a carboxyl group-containing polymer composition was obtained as a white fine powder in the same manner as in Example 1 except that the timing of adding the nonionic surfactant (B) was changed to the time when the polymerization degree was 5% (the time when the temperature reached 60 to 65°C).

(Example 4)

Following the procedure of Example 1, 38 g of a carboxyl group-containing polymer composition was obtained as a white fine powder in the same manner as in Example 1 except that the timing of adding the nonionic surfactant (B) was changed to the time when the polymerization degree was 68% (the time when the flask had been kept at 60 to 65°C for about 2 hours).

(Reference example 5)

Following the procedure of Example 1, 38 g of a carboxyl group-containing polymer composition was obtained as a white fine powder in the same manner as in Example 1 except that the timing of adding the nonionic surfactant (B) was changed to the time when the polymerization degree was 75% (the time when the flask had been kept at 60 to 65°C for about 2.5 hours).

(Example 6)

Following the procedure of Example 1, 38 g of a carboxyl group-containing polymer composition was obtained as a white fine powder in the same manner as in Example 1 except that Blemmer VMA-70 was not used. The polymerization degree when the nonionic surfactant was added was 54%.

(Comparative Example 1)

Following the procedure of Example 1, 38 g of a carboxyl group-containing polymer composition was obtained as a white fine powder in the same manner as in Example 1 except that the timing of adding the nonionic surfactant (B) was changed to the time when the polymerization degree was 0% (the time when the monomers and the like were introduced).

(Comparative Example 2)

Following the procedure of Example 1, 38 g of a carboxyl group-containing polymer composition was obtained as a white fine powder in the same manner as in Example 1 except that the timing of adding the nonionic surfactant (B) was changed to the time when the polymerization degree was 85% (the time when the monomers and the like were introduced).

(Comparative Example 3)

Following the procedure of Example 1, 38 g of a carboxyl group-containing polymer composition was obtained as a white fine powder in the same manner as in Example 1 except that the timing of adding the nonionic surfactant (B) was changed to the time after the completion of polymerization (the time when the polymerization degree was 100%).

[Evaluation Results]

The various characteristics of the carboxyl group-containing polymer compositions obtained in the manners described in Examples 1 to 6, and Comparative Examples 1 to 3 were measured and evaluated using the above-described methods.

Example 5 is a reference example

The results are shown in Table 1.

[Table 1]

| | | Nonionic surfactant | | Polymerization degree during addition | Dispersion time in water (minute) | Viscosity of alcohol gel A [mPa·s] | Light transmittance of alcohol gel A [%] | Odor | |
|---|---|---|---|---|---|---|---|---|---|
| | | Type | HLB value | | | | | Monitor evaluation (score) | Residual hexane [ppm] |
| Example | 1 | Block copolymer of 12-hydroxystearic acid and ethylene oxide | 6 | 54% | 10 | 9940 | 96 | 3.5 | 510 |
| | 2 | Polyoxyethylene (30) hydrogenated castor oil triisostearate | 7 | 50% | 10 | 5000 | 97 | 3.6 | 520 |
| | 3 | Block copolymer of 12-hydroxystearic acid and ethylene oxide | 6 | 5% | 15 | 8540 | 96 | 3.9 | 430 |
| | 4 | Block copolymer of 12-hydroxystearic acid and ethylene oxide | 6 | 68% | 12 | 10320 | 97 | 3.3 | 720 |
| | 5 | Block copolymer of 12-hydroxystearic acid and ethylene oxide | 6 | 75% | 11 | 11000 | 95 | 3.2 | 970 |
| | 6 | Block copolymer of 12-hydroxystearic acid and ethylene oxide | 6 | 54% | 10 | 5420 | 95 | 3.4 | 560 |
| Comparative Example | 1 | Block copolymer of 12-hydroxystearic acid and ethylene oxide | 6 | 0% | 12 | 3000 | 96 | 4.1 | 400 |
| | 2 | Block copolymer of 12-hydroxystearic acid and ethylene oxide | 6 | 85% | 12 | 14000 | 93 | 2.8 | 1520 |
| | 3 | Block copolymer of 12-hydroxystearic acid and ethylene oxide | 6 | 95% | 10 | 14000 | 92 | 2.2 | 2100 |

Table 1 shows that the carboxyl group-containing polymer compositions obtained by adding the nonionic surfactant when the polymerization degree of the $\alpha,\beta$-unsaturated carboxylic acid (a-1) is 5% or more to less than 70% (Examples 1 to 4 and 6) have little odor, excellent dispersibility in water, and excellent transparency and excellent thickening properties for alcohol gels. In particular, in the carboxyl group-containing polymer compositions obtained by adding the nonionic surfactant when the polymerization degree of the $\alpha,\beta$-unsaturated carboxylic acid (a-1) was 5% or more and less than 70% (Examples 1 to 4, and 6), the residual hexane (residual normal hexane) was particularly less.

## Claims

1.   A carboxyl group-containing polymer composition comprising:

a carboxyl group-containing polymer (A) that is a copolymer of monomers containing an $\alpha,\beta$-unsaturated carboxylic acid (a-1) and a compound (a-2) having at least two ethylenically unsaturated groups in its molecule; and
a nonionic surfactant (B) having an HLB value of 5 to 8,
wherein the carboxyl group-containing polymer composition is produced by a method comprising a step of copolymerizing the monomers, wherein the nonionic surfactant (B) is added into a system when a polymerization degree of the $\alpha,\beta$-unsaturated carboxylic acid (a-1) reaches 5% or more to less than 70% in the step, and
wherein an alcohol gel A prepared by the following preparation method has a viscosity of 5,000 mPa s or more at 25°C, as measure by the method described in the description,
the alcohol gel A has a light transmittance of 95% or more at a wavelength of 425 nm, as measure by the method described in the description, and
the preparation method of the alcohol gel A includes homogeneously mixing 0.75 parts by mass of the carboxyl group-containing polymer composition, 51.70 parts by mass of ion exchange water, and 96.0 parts by mass of ethanol with each other, and then adding an ethanol solution of triethanolamine having a concentration of 50% by mass to the resulting mixture until a pH of the mixture reaches 7.0, followed by stirring until the mixture becomes homogeneous to prepare the alcohol gel A.

2.   The carboxyl group-containing polymer composition according to claim 1, wherein a content of the nonionic surfactant (B) is 0.5 to 9 parts by mass with respect to 100 parts by mass of the $\alpha,\beta$-unsaturated carboxylic acid (a-1).

3.   A method for producing a carboxyl group-containing polymer composition containing a carboxyl group-containing polymer (A) that is a copolymer of monomers containing an $\alpha,\beta$-unsaturated carboxylic acid (a-1) and a compound (a-2) having at least two ethylenically unsaturated groups in its molecule, and a nonionic surfactant (B) having an HLB value of 5 to 8, the method comprising a step of copolymerizing the monomers, wherein the nonionic surfactant (B) is added into a system when a polymerization degree of the $\alpha,\beta$-unsaturated carboxylic acid (a-1) reaches 5 or more to less than 70% in the step.

4.   The method for producing a carboxyl group-containing polymer composition according to claim 3, wherein a content of the nonionic surfactant (B) is 0.5 to 9 parts by mass with respect to 100 parts by mass of the $\alpha,\beta$-unsaturated carboxylic acid (a-1).

## Patentansprüche

1.   Carboxylgruppen-hältige Polymer-Zusammensetzung, die Folgendes umfasst:

ein Carboxylgruppen-hältiges Polymer (A), das ein Copolymer von Monomeren ist, die eine $\alpha,\beta$-ungesättigte Carbonsäure (a-1) und eine Verbindung (a-2) mit zumindest zwei ethylenisch ungesättigten Gruppen im Molekül umfassen; und
ein nichtionisches Tensid (B) mit einem HLB-Wert von 5 bis 8,
wobei die Carboxylgruppen-hältige Polymer-Zusammensetzung durch ein Verfahren hergestellt wird, das einen Schritt des Copolymerisierens der Monomere umfasst, wobei das nichtionische Tensid (B) zu dem System zugesetzt wird, wenn der Polymerisationsgrad der $\alpha,\beta$-ungesättigten Carbonsäure (a-1) in dem Schritt 5 % oder mehr bis weniger als 70 % erreicht, und
wobei ein Alkoholgel A, das durch das folgende Herstellungsverfahren hergestellt wurde, eine durch das in der Beschreibung beschriebene Verfahren gemessene Viskosität von 5.000 mPa.s oder mehr bei 25 °C aufweist, das Alkoholgel A eine durch das in der Beschreibung beschriebene Verfahren gemessene Lichtdurchlässigkeit von 95 % oder mehr bei einer Wellenlänge von 425 nm aufweist und

das Herstellungsverfahren des Alkoholgels A das homogene Miteinander-Vermischen von 0,75 Massenteilen der Carboxylgruppen-hältigen Polymer-Zusammensetzung, 51,70 Massenteilen eines Ionenaustauschwassers und 96,0 Massenteilen Ethanol und das anschließende Zusetzen einer Ethanol-Lösung von Triethanolamin mit einer Konzentration von 50 Masse-% zum resultierenden Gemisch umfasst, bis der pH des Gemischs 7,0 erreicht, gefolgt von Rühren, bis das Gemisch homogen wird, um das Alkoholgel A herzustellen.

**2.** Carboxylgruppen-hältige Polymer-Zusammensetzung nach Anspruch 1, wobei der Gehalt des nichtionischen Tensids (B) 0,5 bis 9 Massenteile in Bezug auf 100 Massenteile der $\alpha,\beta$-ungesättigten Carbonsäure (a-1) beträgt.

**3.** Verfahren zur Herstellung einer Carboxylgruppen-hältigen Polymer-Zusammensetzung, die ein Carboxylgruppen-hältiges Polymer (A), das ein Copolymer von Monomeren ist, die eine $\alpha,\beta$-ungesättigte Carbonsäure (a-1) und eine Verbindung (a-2) mit zumindest zwei ethylenisch ungesättigten Gruppen im Molekül umfassen, und ein nichtionisches Tensid (B) mit einem HLB-Wert von 5 bis 8 enthält, wobei das Verfahren einen Schritt des Copolymerisierens der Monomere umfasst, wobei das nichtionische Tensid (B) zu dem System zugesetzt wird, wenn der Polymerisationsgrad der $\alpha,\beta$-ungesättigten Carbonsäure (a-1) in dem Schritt 5 oder mehr bis weniger als 70 % erreicht.

**4.** Verfahren zur Herstellung einer Carboxylgruppen-hältigen Polymer-Zusammensetzung nach Anspruch 3, wobei der Gehalt des nichtionischen Tensids (B) 0,5 bis 9 Massenteile in Bezug auf 100 Massenteile der $\alpha,\beta$-ungesättigten Carbonsäure (a-1) beträgt.

## Revendications

**1.** Composition de polymère contenant un groupe carboxyle comprenant :

un polymère contenant un groupe carboxyle (A) qui est un copolymère de monomères contenant un acide carboxylique $\alpha,\beta$-insaturé (a-1) et un composé (a-2) présentant au moins deux groupes éthyléniquement insaturés dans sa molécule ; et

un tensioactif non ionique (B) présentant une valeur HLB de 5 à 8,

dans laquelle la composition de polymère contenant un groupe carboxyle est produite par un procédé comprenant une étape de copolymérisation des monomères, dans laquelle le tensioactif non ionique (B) est ajouté dans un système lorsqu'un degré de polymérisation de l'acide carboxylique $\alpha,\beta$-insaturé (a-1) atteint de 5 % ou plus à moins de 70 % dans l'étape, et

dans laquelle un gel d'alcool A préparé par le procédé de préparation suivant présente une viscosité de 5 000 mPa•s ou plus à une température de 25°C, telle que mesurée par le procédé décrit dans la description,

le gel d'alcool A présente une transmittance de lumière de 95 % ou plus à une longueur d'onde de 425 nm, telle que mesurée par le procédé décrit dans la description, et

le procédé de préparation du gel d'alcool A consiste à mélanger de manière homogène 0,75 partie en masse de la composition de polymère contenant un groupe carboxyle, 51,70 parties en masse d'eau échangeuse d'ions et 96,0 parties en masse d'éthanol, puis à ajouter au mélange résultant une solution d'éthanol de triéthanolamine présentant une concentration de 50 % en masse jusqu'à ce que le pH du mélange atteigne 7,0, puis à agiter jusqu'à ce que le mélange devienne homogène pour préparer le gel d'alcool A.

**2.** Composition de polymère contenant un groupe carboxyle selon la revendication 1, dans laquelle une teneur du tensioactif non ionique (B) est de 0,5 à 9 parties en masse par rapport à 100 parties en masse de l'acide carboxylique $\alpha,\beta$-insaturé (a-1).

**3.** Procédé de production d'une composition de polymère contenant un groupe carboxyle contenant un polymère contenant un groupe carboxyle (A) qui est un copolymère de monomères contenant un acide carboxylique $\alpha,\beta$-insaturé (a-1) et un composé (a-2) présentant au moins deux groupes éthyléniquement insaturés dans sa molécule, et un tensioactif non ionique (B) présentant une valeur HLB de 5 à 8, le procédé comprenant une étape de copolymérisation des monomères,

dans lequel le tensioactif non ionique (B) est ajouté dans un système lorsqu'un degré de polymérisation de l'acide carboxylique $\alpha,\beta$-insaturé (a-1) atteint de 5 ou plus à moins de 70 % dans l'étape.

**4.** Procédé de production d'une composition de polymère contenant un groupe carboxyle selon la revendication 3, dans lequel une teneur du tensioactif non ionique (B) est de 0,5 à 9 parties en masse par rapport à 100 parties en masse de l'acide carboxylique $\alpha,\beta$-insaturé (a-1).

**EP 3 680 293 B1**

**Patent documents cited in the description**

- JP 2000355614 A
- EP 3205694 A1
- JP 2011105833 A